# EUROPEAN PATENT SPECIFICATION

(11) **EP 1 745 769 B1**
(45) Date of publication and mention of the grant of the patent: **15.10.2008**
(21) Application number: 05015934.2
(22) Date of filing: 22.07.2005
(51) Int. Cl.: A61K 8/04, A61Q 5/06

(54) **Hair treatment method using dry foam as mechanical support for hair**
Haarbehandlungsmethode mit einem trockenem Schaum, der als ein mechanischer Träger verwendet wird
Méthode de traitement capillaire utilisant une mousse sèche pour supporter mécaniquement les cheveux.

(43) Date of publication of application: 24.01.2007
(73) Proprietor: Wella Aktiengesellschaft, 64274 Darmstadt (DE)
(72) Inventor: Kalbfleisch, Axel, 64295 Darmstadt (DE); Rehmann, Andre, 3185 Schmitten (CH); Maillefer, Sarah, Dr., 1749 Middes (CH); Plüss, Jolanda, 1700 Fribourg (CH); Baumeister, Jan, Dr., 1726 Farvagny-le-Grand (CH)

(56) References cited:
- EP-A- 0 247 608
- EP-A- 0 796 611
- WO-A-95/13788
- WO-A-02/087520
- US-A- 3 912 665
- US-A- 6 106 849
- US-A1- 2002 086 038
- US-A1- 2004 028 632
- DATABASE CA CHEMICAL ABSTRACTS SERVICE, COLUMBUS, OHIO, US; 2003, XP002360075 retrieved from STN Database accession no. 138: 142170 & JP 2003 034621 A (LION CORP.) 7 February 2003 (2003-02-07)
- DATABASE CA CHEMICAL ABSTRACTS SERVICE, COLUMBUS, OHIO, US; 2004, XP002360076 retrieved from STN Database accession no. 142:11199 & KR 147 889 A (CHEIL FOODS & CHEMICALS LTD) 17 August 1998 (1998-08-17)

## Description

### FIELD OF THE INVENTION

The present invention relates to a cosmetic method of treating hair comprising formation and application of a dried foam wherein the foam is applied or generated between the scalp and the hair and thereby raising the hair. The methods comprise hair styling methods, methods of permanently shaping the hair, methods of coloring hair and hair care methods. The invention also relates to devices for use in said method.

### BACKGROUND OF THE INVENTION

It is often necessary to stabilize part of the hair, e.g. certain hair strands in specific positions during hair treatment or scalp treatment methods such as for example styling, permanently waving, straightening, coloring, bleaching or conditioning of hair. Conventional mechanical hair styling aids used for this purpose are curlers, rollers, pins, clips or hairgrips. These mechanical aids must be attached in the hair prior to the treatment method and they must be removed when the treatment is finished. The use of these mechanical aids for temporarily holding the hair in place is often tedious and awkward for the consumer or for the hair dresser and the mechanical aids themselfes do not have any additional beneficial effect for conditioning hair or skin. Furthermore, mechanical aids like curlers or rollers have fixed geometrical dimensions. Hair curling methods using these curlers or rollers are rather inflexible in that they lead to hair curles only of a single, fixed shape, predetermined by the diameter of the curler or roller.

A method for styling hair comprising the step of applying a stable foam to hair avoiding the need for rollers etc. is disclosed in US 4,299,240. The method comprises the steps of applying a stable foam, capable of retaining its form upon manipulation, to clean, damp hair to form a pliable mass of hair and foam; shaping the pliable mass in sections, each section being retained, unassisted by mechanical styling aids, in a determined sculptured shape by the stable foam; drying the shaped pliable mass, the pliable mass becoming set in a semi-rigid sculptured shape upon drying the foam; and brushing the semi-rigid hair to finally shape the hair and to remove the foam residue. The foams disclosed in US 4,299,240 are based on lauryl sulfate and gelatin and have only a limited stability because they are only stable in the wet state. They are described to dissipate upon drying. Thus, they cannot form dry foams. Espcecially when exposed to the higher temperatures of a hair dryer, the foam will quickly dissipate. Furthermore, the hair is an integral part of the pliable mass and is contained within the mass. It is not possible to treat the hair with a different composition without destroying the pliable mass. And it is not possible with this method to apply the foam between scalp and scalp hair and thereby raising or lifting single hair strands to selectively treat other hair strands beneath or to selectively treat the underlying scalp. The wet foam of the prior art is not strong enough to support itself and hair lying untop of it but needs hair to be incorporated within a mass of hair and foam and the hair is needed for supporting the stability of the mass.

Therefore, a need exists for substitutes for the conventional mechanical hair styling aids that can be easily applied, easily removed, can lead to a wide variety of shapes and curl geometries when used in a hair shaping method and can have additional conditioning effects on hair or skin besides their primary function of temporarily raising hair to a specific position and which have an improved stability and do not collapse or dissipate when dried or used in methods employing heating devices such as hair driers. Another object of the invention is to provide a method for increasing the volume of tthe hairstyle.

### SUMMARY OF THE INVENTION

The present invention is directed to a cosmetic treatment method comprising
- providing a foamable composition,
- forming a foam from said foamable composition on release from a pressurized container,
- drying said foam wherein the foam does not disappear upon drying,
- wherein said foam is applied or generated between the scalp and scalp hair and thereby raising the hair.
Said composition comprises
(a) at least 1% by weight based on the composition without propellants of a zwitterionic surfactant or a surfactant mixture comprising at least one zwitterionic surfactant.
(b) at least one hair fixing polymer,
(c) water, and
(d) at least 20% by weight, based on the total composition of at least one propellant.

The present invention is further directed to a device comprising
- a composition capable of forming a foam that does not disappear on drying; and
- an aerosol container equipped with a foaming device;
wherein said composition comprises
(a) at least 1% by weight based on the composition without propellants of a zwitterionic surfactant or a surfactant mixture comprising at least one zwitterionic surfactant,
(b) at least one hair fixing polymer,
(c) water, and
(d) at least 20% by weight, based on the total composition of at least one propellant and

The present invention is further directed to the use of said device in a method according to the invention and to the use of dry foam in a cosmetic method of treating scalp hair or the scalp.

These and other features, aspects, and advantages of the present invention will become evident to those skilled in the art from a reading of the present disclosure.

### DETAILED DESCRIPTION OF THE INVENTION

The cosmetic treatment method of the present invention includes the use of a foamable composition which forms a foam when released from a pressurized container wherein the foam hardens upon drying, i.e. the foam does not disappear, dissipate or collapse when the volatile components of the foamable composition such as water or alcohol evaporate under ambient conditions or upon drying with a heating device such as a hair drier.

A foam is a system of gas filled spherical or polyhedral cells with boundaries of liquid, semi-liquid, highly viscous or solid cell walls or lamellae. The cell walls or lamellae are connected via struts and intersections to form a connected framework. In closed cell foams there are lamellae between the struts. In open cell foams the lamellae are destroyed but with a framework of interconnected struts in place. A foam according to the invention can be open celled or closed celled. A foamable composition is a composition capable of forming a foam when a gas is introduced. A hardenable foam is a wet foam that can be dehydrated to a dried, hardened foam. A dry foam according to the invention is a dehydrated foam that does not collapse but forms a spherical or polyhedral agglomerate of open or closed bubbles with a rigid closed cell or open cell framework of interconnected struts of solid material, such as solid surfactants or polymers.

Each of the components of the foamable composition, as well as preferred or optional components, is described in detail hereinafter. All percentages, parts and ratios are based upon the total weight of the composition without propellant, unless otherwise specified. The amount of the propellant is based on the total weight of the composition including propellant. All such weights as they pertain to listed ingredients are based on the active level and, therefore, do not include solvents or by-products that may be included in commercially available materials, unless otherwise specified. All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods of the present invention can comprise, consist of, and consist essentially of the essential elements and limitations of the invention described herein, as well as any of the additional or optional ingredients, components, steps, or limitations described herein.

The term "polymer" as used herein shall include materials whether made by polymerization of one type of monomer (homopolymers) or made by two or more types of monomers (copolymers). The term "water soluble" as used herein, means that the compound is soluble in water in the present composition. In general, the compound should be soluble at 25 °C at a concentration of 0.1% by weight of the water solvent, preferably at 1%, more preferably at 5%, most preferably at 15%. The term "water dispersible" as used herein, means that the compound is dispersible in water in the present composition to form a homogeneous dispersion with or without dispersing aids.

All cited references are incorporated herein by reference in their entireties. Citation of any reference is not an admission regarding any determination as to its availability as prior art to the claimed invention.

The device of the present invention includes a composition capable of forming a foam that can be dried without dissipating; and an aerosol container equipped with a foaming device. The hardenable foam forming capability of the composition can be ensured by a content of a combination of effective amounts of surfactants and polymers in an aqueous medium. Surfactants and polymers can interact to support the solid foam structure after drying.

### Surfactants

The amount of surfactant is at least 1% by weight, e.g. 1 to 15% by weight, more preferred at least 2% by weight, e.g. 2 to 10% by weight. The foamable composition of the invention comprises at least 1% by weight based on the composition without propellants of a zwitterionic surfactant or a surfactant mixture comprising at least one zwitterionic surfactant. Zwitterionic surfactants are surfactants having a positiv charge and a negative charge and a hydrophobic group. Preferred hydrophobic groups are hydrocarbon groups or fatty alkyl groups with 4 to 30 carbon atoms.

Zwitterionic surfactants can be selected from aliphatic quaternary ammonium, phosphonium or sulfonium compounds, substituted with anionic groups such as carboxylate, sulfate, phosphonate oder phosphate groups. They can have the general formula

R¹-Y⁽⁺⁾(R²)ₓ-CH₂-R³-Z⁽⁻⁾

wherein R1 is a linear or branched alkyl-, alkenyl-, or hydroxyalkyl group with 4 to 30, preferably 8 to 18 carbon atoms and 0 to 10 ethylenoxide units and 0 to 1 glycerol units or R¹ is an acylamidoalkyl group such as for example R³-CONH-(CH₂)ₓ- with R³ being an organic group with 8 to 30 carbon atoms, preferably a hydrocarbon group, more preferably an alkyl group to 30 carbon atoms; Y is an N-, P- oder S-atom; R2 is an alkyl or mono-hydroxyalkyl group with preferably 1 to 3 carbon atoms; x is 1 if Y is a sulfur atom and x is 2 if Y is a nitrogen or phosphor atom; R3 is an alkyl- or hydroxyalkyl group with preferably 1 to 4C-Atomen and Z is a carboxylate-, sulfate-, phosphonate- or phosphate group.

Preferred zwitterionic surfactants are those of general formula

R¹-N⁽⁺⁾(R²)₂-A-Z⁽⁻⁾

wherein R¹ is an organic group with 8 to 30 carbon atoms, preferably a hydrocarbon group, more preferably an alkyl group or R¹ is an acylamidoalkyl group such as for example R³-CONH-(CH₂)ₓ- with R³ being an organic group with 8 to 30 carbon atoms, preferably a hydrocarbon group, more preferably an alkyl group to 30 carbon atoms; x is a number of 1 to 4; R² is an organic group with 1 to 4 carbon atoms, preferably a hydrocarbon group, more preferably an alkyl or an hydroxyalkyl group; A is a divalent organic group with 1 to 10 carbon atoms, preferably a hydrocarbon group, more preferably an alkylene or hydroxyalkylene group; and Z⁽⁻⁾ is COO⁽⁻⁾ (as in betaines) or SO₃⁽⁻⁾ (as in sultaines).

Preferred zwitterionic surfactants are those containing a betaine structural unit, e.g. C8- to C18-alkylbetaine such as cocodimethylcarboxymethyl betaine, lauryldimethyl carboxymethyl betaine, lauryldimethyl-alpha-carboxyethyl betaine, cetyldimethyl carboxymethyl betaine, oleyldimethyl-gamma-carboxypropyl betaine or lauryl-bis-(2-hydroxy-propyl)-alpha-carboxyethyl betaine; C8- to C18-alkyl sulfobetaine such as cocodimethyl sulfopropylbetain, stearyldimethyl sulfopropylbetain, lauryldimethyl sulfoethylbetain, lauryl-bis-(2-hydroxyethyl) sulfopropylbetain; carboxyl substituted imidazols, C8- to C18-alkyldimethylammonium acetate, C8- to C18-alkyldimethyl carbonylmethyl ammonium salts such as C8- to C18-fatty acid alkylamido betaine such as cocofatty acid amidopropyl betaine (INCI-name: Cocamidopropylbetaine) and N-cocofatty acid amidoethyl-N-[2-(carboxymethoxy)ethyl] glycerol (INCI-name: Disodium Cocoamphodiacetate).

Other preferred zwitterionic surfactants contain a sultaine structure such as alkyl sultaines and fatty acid amidoalkyl hydroxysultaines, e.g. Cocamidopropyl Hydroxysultaine, Coco-Hydroxysultaine, Coco-Sultaine, Erucamidopropyl Hydroxysultaine, Lauramidopropyl Hydroxysultaine, Lauryl Hydroxysultaine, Lauryl Sultaine, Myrist-amidopropyl Hydroxysultaine, Oleamidopropyl Hydroxysultaine, Tallowamidopropyl Hydroxysultaine. Most preferred is Cocamidopropyl Hydroxysultaine.

### Hair fixing polymer

The composition of the invention comprises at least one hair fixing polymer. The amount of hair fixing polymer is preferably at least 1% by weight, e.g. from 1 to 20, or from 2 to 18, or from 5 to 15% by weight based on the total composition without propellant. The hair fixing polymer can be nonionic, anionic, cationic, amphoteric or zwitterionic, preferably it is nonionic. The hair fixing polymer can be synthetic or natural. The term "natural polymer" also comprises chemically modified polymers of natural origin. Preferred are polymers which are soluble in the aqueous or aqueous-alcoholic carrier. Hair fixing polymers are polymeric compounds which impart hair-holding or style-retention properties to hair, e.g. when applied as 0,01 to 5% by weight aqueous, alcoholic or aqueous-alcoholic solution or dispersion. In particular, hair fixing polymers are those polymers listed in the International Cosmetic Ingredient Dictionary and Handbook, 10^{th} edition 2004 with the function "Hair Fixatives".

Suitable synthetic, non-ionic hair fixing polymers are for example: homo- oder copolymers of at least one monomer selected from vinyl pyrrolidone; vinyl caprolactam; vinyl ester (e.g. vinyl acetate), vinyl alcohol, acrylamide, methacrylamide, alkyl- and dialkyl acrylamide, alkyl- und dialkyl methacrylamide, dialkylaminoalkyl methacrylamide, dialkylaminoalkyl acrylamide, alkylacrylate, alkylmethacrylate, propylene glycol or ethylene glycol, wherein preferred alkyl groups of these monomers are C1- to C7-alkyl groups, more preferred C1- to C3-alkyl groups. Suitable are e.g. homopolymers of vinyl caprolactam, homopolymers of vinyl pyrrolidone, homopolymers of N-vinyl formamide. Suitable hair fixing polymers are also copolymers of vinyl pyrrolidone and vinyl acetate; terpolymers of vinyl pyrrolidone, vinyl acetate and vinyl propionate; terpolymers of vinyl pyrrolidone, vinyl caprolactam and dialkylaminoalkyl (meth)acrylate; terpolymers of vinyl pyrrolidone, vinyl caprolactam and dialkylaminoalkyl (meth)acrylamide; polyacrylamide; polyvinyl alcohol; and hair fixing polyethylen glycol/polypropylen glycol copolymers. Preferred are nonionic vinyl lactam homo- or copolymers. Suitable vinyl lactams are e.g. vinyl caprolactam and vinylpyrrolidone. Especially preferred are polyvinyl pyrrolidone, polyvinyl caprolactam and polyvinyl pyrrolidone/vinyl acetate copolymers which are marketed e.g as Luviskol® VA 37 and Luviskol® VA 64.

Suitable synthetic, anionic hair fixing polymers can be synthetic or natural homo- or copolymers from monomeric units with acid groups. The monomers with acid groups can be copolymerised with monomers without acid groups. Preferred acid groups are -COOH, -SO₃H, -OSO₃H, -OPO₂H und -OPO₃H₂, carboxylic acid being most preferred. The acid groups can be unneutralised, partially neutralised or completely neutralised. Preferred is a degree of neutralisation of from 50 to 100%. Suitable monomers are ethylenically unsaturated, radically polymerisable compounds carrying at least one acid group, e.g. styrene sulfonic acid, 2-acrylamide-2-methylpropane sulfonic acid or carboxyvinyl monomers like acrylic acid, methacrylic acid, crotonic acid, fumaric acid, maleic acid, maleic anhydride and its monoesters or itaconic acid.

Comonomers without acid groups are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester, vinyl alcohol, propylen glycol or ethylen glycol, amine substituted vinyl monomers such as dialkylaminoalkyl acrylate, dialkylaminoalkyl methacrylate, monoalkylaminoalkyl acrylate and monoalkylaminoalkyl methacrylate, wherein preferred alkyl groups are C1- to C7-alkyl groups, especially C1- to C3-alkyl groups.

Suitable anionic hair fixing polymers are in particular copolymers of acrylic or methacrylic acid with monomers selected from acrylic acid esters, methacrylic acid esters, acrylamides, methacrylamides and vinyl pyrrolidone; homopolymers of crotonic acid; copolymers of crotonic acid with monomers selected from vinyl esters, acrylic acid esters, methacrylic acid esters, acrylamides, methacrylamides. A natural anionic hair fixing polymer is shellac. Preferred anionic hair fixing polymers are vinylacetate/crotonic acid copolymer; partially esterified copolymers of vinyl methylether and maleic anhydride; terpolymers of acrylic acid, alkyl acrylate and N-alkyl acrylamide, e.g. acrylic acid/ethyl acrylate/N-t-butyl acrylamide terpolymer; terpolymers of vinyl acetate, crotonic acid and vinyl alkanoate, e.g. vinyl acetate/crotonic acid/vinyl neodecanoate copolymer.

Suitable synthetic, amphoteric hair fixing polymers are polymers with anionic or acidic functional groups as well as cationic or basic functional groups. The acidic or anionic functional groups are those as definied above for the anionic polymers. Cationic or basic functional groups are in particular primary, secondary or tertiary amine groups or quaternary ammonium groups. Preferred examples are copolymers of alkyl acrylamide (especially octyl acrylamide), alkylaminoalkyl methacrylate (especially t-butylaminoethyl methacrylate) and two or more monomers selected from acrylic acid, methacrylic acid and their esters, wherein the alkylgroups have from 1 to 4C-atoms and at least one of the monomers has an acid group. A marketed product is e.g. Amphomer® oder Amphomer® LV-71 of National Starch. Further examples for hair fixing polymers are copolymers of acrylic acid, methyl acrylate and methacrylamidopropyl trimethylammonium chloride (INCI-name: polyquaternium-47); copolymer of acrylamidopropyl trimethylammonium chloride and acrylates; or copolymers of acrylamide, acrylamidopropyl trimethylammonium chloride, 2-amidopropyl acrylamide sulfonate and dimethylaminopropyl amine (INCI-name: polyquatemium-43). Suitable are also polymers with betaine groups, e.g. copolymers of methacryloyl ethylbetaine and two or more monomers selected from acrylic acid and its alkyl esters (INCI-name Methacryloyl Ethyl Betaine/Acrylates Copolymer).

Suitable cationic hair fixing polymers are polymers with cationic or basic functional groups. Cationic or basic functional groups are in particular primary, secondary or tertiary amine groups or quaternary ammonium groups. The cationic charge density is preferably from 1 to 7 meq/g. The cationic polymers can be homopolymers or copolymers wherein the cationic or basic functional group can be part of the polymeric backbone or can be a pendant group. Monomers with cationic or basic groups can be copolymerised with monomers without cationic or basic group.

Suitable cationic monomers are ethylenically unsaturated radically polymerisable compounds with at least one cationic or basic group, e.g. ammonium substituted vinyl monomers such as trialkyl methacryloxy alkylammonium, trialkyl acryloxy alkyl ammonium, dialkyl diallyl ammonium and quaternary vinyl ammonium monomers with cyclic nitrogen containing groups such as pyridinium, imidazolium or quaternary pyrrolidones, e.g. alkylvinyl imidazolium, alkylvinyl pyridinium. The alkyl groups of these monomers are preferably lower alkyl groups such as C1 to C7 alkyl groups, more preferred C1 to C3 alkyl groups. The cationic monomers can be polymerised with non-cationic comonomers. Non-cationic comonomers are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester such as vinyl acetate, vinyl alcohol, propylen glycol or ethylen glycol, wherein preferred alkyl groups are C1- to C7-alkyl groups, especially C1- to C3-alkyl groups.

Suitable cationic hair fixing polymers are for examples those listed in the International Cosmetic Ingredient Dictionary and Handbook, 10^{th} edition 2004 as polyquatemium, e.g. methylvinyl imidazolium chloride/vinyl pyrrolidone copolymer (Polyquaternium-16) or quaternised vinyl pyrrolidone/dimethylaminoethyl methacrylate copolymer (Polyquaternium-11). Preferred synthetic cationic hair fixing polymers are: poly(dimethyl diallyl ammonium chloride); copolymers of acrylamide and dimethyl diallyl ammonium chloride; quaternary ammonium polymers made by reaction of diethylsulfate and a copolymer of vinyl pyrrolidone and dimethylaminoethyl methacrylate (e.g. GAFQUAT® 755 N, GAFQUAT® 734); quaternary ammonium polymers of methylvinyl imidazolium chloride and vinyl pyrrolidone (e.g. LUVIQUAT® HM 550); Polyquaternium-35; Polyquaternium-57; polymer of trimethylammoniumethyl methacrylate chloride; terpolymer of dimethyl diallyl ammonium chloride, sodium acrylate and acrylamide (e.g. MERQUAT® Plus 3300); copolymer of vinyl pyrrolidone, dimethylaminopropyl methacrylamide and methacryloyl aminopropyllauryl dimethyl ammonium chloride; terpolymer of vinylpyrrolidone, dimethylaminoethyl methacrylate and vinyl caprolactam (e.g. GAFFIX® VC 713); vinyl pyrrolidone / methacryl amidopropyl trimethylammonium chloride copolymer (e.g. GAFQUAT® HS 100); copolymer of vinyl pyrrolidone and dimethylaminoethyl methacrylate; copolymer of vinyl pyrrolidone, vinyl caprolactam and dimethylaminopropyl acrylamide; poly- or oligoester made of at least one monomer selected from hydroxyacids which are substituted with at least one quaternary ammonium group.

Suitable cationic polymers derived from natural polymers are for example cationic derivatives of polysaccharides such as cationic derivatives of cellulose, starch or guar. Suitable are also chitosan and chitosan derivatives. Cationic polysaccharides have for example the general formula

G-O-B-N⁺R^{a}R^{b}R^{c} X⁻

Wherein G is an anhydroglucose group such as starch anhydroglucose or cellulose anhydroglucose; B is a divalent bridging group such as alkylen, oxyalkylen, polyoxyalkylen or hydroxyalkylen; R^{a}, R^{b} and R^{c} are independent from one another alkyl, aryl, alkylaryl, arylalkyl, alkoxyalkyl or alkoxyaryl with each up to 18 carbon atoms, wherein the total number of carbon atoms in R^{a}, R^{b} and R^{c} is preferably a maximum of 20; X is a counter ion, such as halogen, acetate, phosphate, nitrate or alkylsulfate, preferably chloride. Cationic cellulose polymers are for example those with the INCI-names Polyquaternium-10 or Polyquaternium-24. A cationic guar derivative is for example that with the INCI-name Guar Hydroxypropyltrimonium Chloride.

Especially preferred cationic hair fixing polymers are chitosan, chitosan salts and chitosan derivatives. Chitosans are totally or partially deacetylated chitines. The molecular weight can be for example from about 20000 to about 5 Millionen g/mol, e.g.. from 30000 to 70000 g/mol for lower molecular weight chitosan. Preferred are high molecular chitosans with a molecular weight above 100000 g/mol, more preferred from 200000 to 700000 g/mol. The degree of deacetylation is preferably from 10 to 99%, more preferred from 60 to 99%. A preferred chitosan salt is chitosonium pyrrolidonecarboxylate, e.g. KYTAMER® PC with a molecular weight of about 200000 to 300000 g/mol and a degree of deacetylation of 70 to 85%. Chitosan derivatives are for example quaternised chitosans, alkylated chitsoans or hydroxyalkylated chitsoans such as hydroxyethyl-, hydroxypropyl- or hydroxybutyl chitosan. The chitosan or chitosan derivatives are preferably partially or completely neutralised. The degree of neutralisation is preferably at least 50%, more preferred from 70 to 100%, based on the total number of amino groups. In principle, all cosmetic acceptable inorganic or organic acids can be used for neutralisation, such as formic acid, tartaric acid, malic acid, lactic acid, citric acid, pyrrolidone carboxylic acid, glycolic acid, hydrochloric acid etc., pyrrolidone carboxylic acid being especially preferred.

Preferred cationic polymers on a natural basis are: cationic cellulose derivatives made from hydroxyethylcellulose and diallyl dimethyl ammonium chloride; cationic cellulose derivatives made from hydroxyethylcellulose and trimethyl ammonium substituted epoxide; chitosan and ist salts; hydroxyalkyl chitosan and ist salts; alkylhydroxyalkyl chitosan and ist salts; N-hydroxyalkyl chitosan alkylether.

Most preferred hair fixing polymers are polyvinylpyrrolidone (INCI-name PVP; trade names e.g. Luviskol® K30, K85, K90 available from BASF); copolymers of vinylpyyrolidone and vinylacetate (INCI-name VP/VA copolymer; trade names e.g. Luviskol® VA37, VA64 available from BASF); copolymers of vinylpyrrolidone, methacrylamide and vinylimidazole (INCI-name VP/Methacrylamide/Vinyl Imidazole Copolymer, trade name Luviset® Clear available from BASF); copolymers of vinylacetate and crotonic acid (INCI-name VA/Crotonates Copolymer, trade name Luviset® CA66 available from BASF); copolymers of octylacrylamide, acrylic acid, butylamino methacrylate, methyl methacrylate and hydroxypropyl methacrylate (INCI-name Octylacrylamide/Acrylates/Butylaminoethyl Methacrylate Copolymer, available from National Starch and Chemical Company); copolymer of alkylacrylate, acrylic acid and alkylacrylamide (INCI-name Acrylates/t-Butylacrylamide Copolymer; trade name Ultrahold® 8 available from BASF); chitosane; methylvinyl imidazolium chloride/vinyl pyrrolidone copolymer; quaternised vinyl pyrrolidone/ dimethylaminoethyl methacrylate copolymer; quaternized cellulose.

### Propellant

The composition of the invention comprises at least one propellant. Amount of propellant is at least 20% by weight, based on the total composition, especially from 20 to 50 or from 25 to 45% by weight. Propellants are for example lower alkanes which are gaseous under ambient conditions such as n-butane, i-butane, propane, or their mixtures; dimethyl ether or fluorinated hydrocarbons, such as 1,1-difluoroethane or tetrafluoroethane or mixtures of these propellants. Other propellants are compressed gases such as air, N₂, N₂O and CO₂ and their mixtures. Hydrocarbons with three to four carbon atoms and their mixtures are especially preferred as the propellant.

### Carrier

The carrier of the composition according to the invention can be aqueous or aqueous-alcoholic. By "aqueous" it is meant that the compositions contain almost only water as solvant, i.e. organic solvants such as C1- to C4 alcohols are not present or they are present only in very minor amounts such as below 2 or below 1% by weight of the total composition. Deionized water is preferably used. Water from natural sources containing mineral cations can also be used, depending on the desired characteristic of the product. By "aqueous-alcoholic" it is meant that the compositions contain significant amounts of water as well as significant amounts of alcoholic solvants. Significant amounts are amounts of e.g. at least 5% by weight or more. The level and species of the carrier are selected according to the compatibility with other components, and other desired characteristics of the product. Alcoholic solvants are organic compounds which are liquid at room temperature (25°). Since alcohols can act as foam inhibitors, the amount of alcohol is kept low enough as not to inhibit foam formation. The amount of alcohol is preferably not more than 20% by weight, preferably 0 to 20% by weight, or from 1 to 15% by weight of the total composition without propellant. Alcohols can be those conventionally used for cosmetic purposes, e.g. monohydric C1 to C6 alcohols such as ethanol and isopropanol. Ethanol is especially preferred. The water content is prerferably from 50 to 95, more preferred from 60 to 90% by weight. An aqueous-alcoholic carrier can contain for example 5 to 20% by weight ethanol and 60 to 90% by weight water, based on the total composition. The pH is preferably in the range of from 6 to 9, more prefarably from 6,5 to 8. Buffers and other pH adjusting agents can be included to achieve or stabilise the desirable pH.

The composition of the invention can additionally contain at least one polyhydric alcohol for further improving the properties. The polyhydric alcohols have at least two alcoholic hydroxyl groups. They have preferably 2 to 6 carbon atoms and 2 to 6 hydroxyl groups such as glycerol, C2- to C4-alkylenglycols, and sorbitol. Especially preferred are glycerol and C2- to C4-alkylenglycols, such as ethylenglycol and propylenglycol. The amount of polyhydric alcohol is preferably from 0,1 to 15, more preferably from 0,5 to 6% by weight based on the total composition without propellant.

### Additives

The composition according to the invention can also contain conventional cosmetic additives usually used in hair treatment compositions, as long as they do not negatively interfere with foam formation, dry foam stability and foam hardening; e.g. fragrances or perfume oils in an amount of 0.01 to 1% by weight; preservatives, such as parabene in an amount of 0.01 to 1% by weight; buffer substances, such as sodium citrate or sodium phosphate, in an amount of 0.1 to 1% by weight; hair care or skin care substances, such as e.g. betaine, panthenol, moisturiser, plant extracts, vegetable extracts, protein hydroylsates and silk hydrolysates, lanolin derivatives, light protective agents, antioxidants, radical-trapping agents, anti-dandruff agents, fatty alcohols, vitamines, direct dye compounds, luster-imparting substances or combability-improving substances in an amount of 0.01 1 to 5% by weight; physiologically compatible silicone derivatives, such as volatile or non-volatile silicone oils or high molecular weight siloxane polymers in an amount of 0.05 to 20% by weight; surfactants and emulsifying agents other than those already mentioned which can be nonionic, anionic, cationic or amphoteric such as ethoxylated fatty alcohols, fatty alcohol sulfates, fatty alcohol ether sulfates, alkylbenzene sulfonate, alkyl trimethylammonium salts in an amount of 0.1 to 15% by weight; product coloring agents in an amount of 0.1 to 1% by weight; pigments in an amount of 0.01 to 25% by weight.

Polymers with acid groups are preferably neutralised up to 50 bis 100%. Nonlimiting examples of neutralising agents include primary or secondary organic amines, or inorganic bases such as ammonia, NaOH, KOH, ammonium hydroxide etc.. Preferred are amino alcohols with 1 to 10 carbon atoms and 1 to 3 hydroxy groups such as aminomethyl propanol (AMP), monethanolamine, diethanol amine, triethanolamine, tetrahydroxypropyl ethylendiamine, diisopropanolamine, tromethamine, and mixtures thereof.

### METHOD OF MAKING

The compositions of the present invention can be made by conventional formulation and mixing techniques.

### Packaging

The composition according to the invention is filled in a pressure-resistant aerosol container or package, which is provided with a device for foam production. The container can be made from any material conventionally used for this purpose such as metal (e.g. aluminium or tinplate can), glass or pressure resistant plastics (e.g. polyethylene terephtalate). The device for foam production can be any known and conventionally used actuator for dispensing foam, foam dispensing head or foam cap.

In a preferred embodiment of the invention, the aerosol container is provided with an elongated nozzle. The elongated nozzle facilitates giving the foam the desired shape or selectively placing the foam at the desired position under the hair, on the scalp or preferably between scalp hair and scalp. The elongated nozzle can be made of any suitable material and can be a tube-shaped extension of the foam head. The preferred length is at least 4 mm or more, e.g. 5 to 100 mm or 10 to 80 mm. The preferred diameter is at least 1 mm or more, e.g. 2 to 20 or 4 to 10 mm. The preferred length to diameter ratio is from 2:1 to 50:1, more preferred from 4:1 to 30:1.

In another preferred embodiment of the invention the aerosol container is provided with a cap or nozzle cap with two or more than two openings. The openings can be in a single line forming a nozzle comb or they can be arranged in more than one line, or in different geometries for example forming a nozzle brush.

### METHOD OF USE

The cosmetic treatment method of the present invention comprises
- providing a foamable composition,
- forming a foam from said foamable composition on release from a pressurized container,
- drying said foam wherein the foam does not disappear upon drying,
wherein said foam is applied or generated between the scalp and scalp hair and thereby raising the hair.

Such method generally involves application of an effective amount of the foamed product to dry, slightly damp, or wet hair, preferably before the hair is arranged to a desired style. By "effective amount" is meant an amount sufficient to provide the hair hold and style benefits desired considering the length of the hair and the desired hair style. In general, from about 0.5g to about 50g of product will be applied to the hair, depending upon the particular product formulation, dispenser type, length of hair, and type of hair style. It is a special advantage of the treatment method of the invention that a raised hair strand can be treated selectively across its length. For example, the hair tips can be selectively treated with hair care agents in a hair care method or can be selectively left untreated or be treated with a lower strength permanent shaping composition during a permanent shaping method. Or different coloring compositions can be applied to different segments of the raised hair strand in a hair coloring method.

In preferred embodiments of the invention, the method comprises performing at least one additional hair treating step, for example
- the method is a non-permanent hair styling method comprising the additional step of bringing the hair in a desired shape; or
- the method is a method for permanently shaping the hair, comprising the additional step of contacting the hair with a permanent shaping composition comprising at least one keratin-reducing agent and/or a fixation composition containing at least one oxidising agent; or
- the method is a method of coloring hair comprising the additional step of contacting the hair with a composition containing a hair coloring agent and/or a hair bleaching agent; or
- the method is a hair care method comprising the additional step of applying a hair care active to the hair.
The hardened foam can be removed by hand or by combing or brushing or by washing or rinsing after performing the additional hair treating step.

### Non-permanent hair styling method

A non-permanent hair styling method according to the invention is a method of temporarily changing the shape of the hair or the hairstyle including changing the volume of the hairstyle, preferably increasing it. A typical hair styling method according to the invention is the following. Freshly shampooed or wet or dry hair is parted e.g. with a comb and a hair strand held upwards. Preferred is an application on wet hair. Dry hair is preferably pre-treated with a conventional hair styling mousse. A foam body of suitable size, length and volume is generated either directly on or near the hair line, or on or near the scalp or is generated seperately and then placed on or near the hair line, or on or near the scalp. The hair strand is positioned on top of the foam body and can optionally be modelled into the desired shape with the hand or a mechanical modelling aid such as a comb. The hair and the foam body are dried, preferably with a drying aid. The drying aid can be an electrical or electromagnetical device such as an hair drier or an infrared device. The foam body is converted to a hardened, solid structure on drying. Finally, the dry foam body is removed, preferably by hand in one or more pieces or it is combed out by a suitable combing device such as a comb or a brush. The curled or waved hair can optionally be treated with an ordinary styling agent such as a hair gel or a hairspray.

### Permanent hair shaping method

A typical permanent hair shaping method according to the invention is the following. Freshly shampooed or wet or dried hair is treated with a permanent shaping composition such as for example a permanent waving or straigthening composition containing at least one permanent shaping agent, preferably at least one keratin reducing agent. The permanent shaping composition is left on the hair for an acting time sufficient for permanent shaping of hair, which depends on hair condition, pH and shaping effectiveness of the shaping composition as well as on the application temperature, amounting for example from 3 to 30 minutes, preferably 2 to 20 minutes. Preferably, the permanent shaping composition is rinsed from the hair with water after the acting time and excessive water is optionally removed from the hair, preferably with a moisture absorbing device such as a towel. The wet hair is parted e.g. with a comb and a hair strand held upwards. A foam body of suitable size, length and volume is generated either directly on or near the hair line, or on or near the scalp or is generated seperately and then placed on or near the hair line, or on or near the scalp. The hair strand is positioned on top of the foam body and can optionally be modelled into the desired shape with the hand or a mechanical modelling aid such as a comb. The hair and the foam body are dried, preferably with a drying aid. The drying aid can be an electrical or electromagnetical device such as an hair drier or an infrared device. The foam body is converted to a hardened, solid structure on drying. Then the hair is treated oxidatively with a fixation composition, preferably a composition containing at least one oxidation agent. Then, the hair is optionally rinsed with water, optionally put into a water wave and dried. The dry foam body is removed, either during the above rinsing step or by hand in one or more pieces or it is combed out by a suitable combing device such as a comb or a brush. The permanently shaped hair can optionally be treated with an ordinary styling agent such as a hair gel or a hairspray or with a conventional hair conditioning composition.

The permanent shaping agents employed in the method described here are based on conventional hair keratin-reducing substances, such as salts of sulfurous acid or mercapto compounds, especially salts or esters of mercaptocarboxylic acids. The hair-shaping composition contains the keratin-reducing compounds in the usual amount for hair shaping, for example the ammonium salts of thioglycolic acid, thiolactic acid or cysteine, in a concentration of from 0,5 to 25% or 1 to 20%, or 5 to 15% or most preferred from 6 to 12% by weight. The pH value of alkaline shaping compositions is typically from 7 to 10. The pH is adjusted preferably with ammonia, monoethanolamine, ammonium carbonate or ammonium hydrogen carbonate. If the shaping component is to be adjusted so that it is acidic (e.g. at pH=6.5 to 6.9), esters of mercaptocarboxylic acid, such as monothioglycolic acid glycol ester or glycerol ester, preferably however mercaptoacetamide or 2-mercaptoproprionic acid amide, are employed in a concentration of preferably 2 to 14% by weight; or the salts of sulfurous acid, for example sodium, ammonium or monoethanol ammonium sulfite, are employed in a concentration of for example 3 to 8% by weight (calculated as SO₂). Preferably the hair keratin-reducing compound employed is a salt or a derivative of a mercaptocarboylic acid. A keratin-reducing compound selected from the group consisting of thioglycolic acid, cysteine, thiolactic acid and their salts is particularly preferred. A swelling and penetrating agent can be added to the permanent hair-shaping component to increase its effectiveness. This swelling and penetrating agent can be urea, multivalent alcohols, ether, melamine, alkali or ammonium thiocyanate, isopropanol, imidazolidin-2-one, 2-pyrrolidone and 1-methyl-2-pyrrolidone. The permanent hair-shaping component preferably contains from about 0.5 to 50% by weight of the swelling and penetrating agent, more preferably from 2 to 30% by weight.

The fixing composition is applied to the hair in an amount of from typically 50 to 200 g, according to the abundance of the hair. Any oxidizing agent currently used in hair fixing compositions can be used in the fixing composition for the hair fixing. For example, potassium bromate, sodium bromate, sodium perborate, dehydro-ascorbic acid, hydrogen peroxide and urea peroxide can be used as the oxidizing agent. The concentration of the oxidizing agent differs according to the application time, usually from 1 to 40 minutes, preferably 5 to 20 minutes, and the application temperature, namely 25 to 50° C. Usually the oxidizing agent is used in a concentration of about 0,1 to 25% or 0,5 to 20% or 2 to 14% or 4 to 12% by weight in the fixing composition. The fixing composition can of course contain other substances, for example weak acids or peroxide stabilizers. The pH-value is preferably from 2 to 6 or 3 to 5, especially when a peroxide is used. The pH-value is preferably from 6 to 9 or 7 to 8,5, especially when a bromate is used. Oxidative agents are for example hydrogenperoxide, urea proxide, bromates, persulfates, perborates, percarbonates, peroxides and iodates, especially alkalibromates such as sodium or potassium bromate, ammonium bromate, alkaline earth metal bromates, alkaline metal persulfates, alkaline earth metal persulfates, ammonium persulfate, alkali metal perborates such as sodium perborate, alkaline earth metal perborates, ammonium perborate, alkali metal percarbonates, alkaline earth metal percarbonates, ammonium percarbonate, calcium peroxide and sodium iodate. Most preferred is hydrogenperoxide. Peroxide stabilizers can be used in amounts of for example 0,01 to 2% or 0,05 to 0,3% by weight. Peroxide stabilizers are for example dialkali hydrogenphosphate such as disodium hydrogenphosphate, p-acetamidophenol, oxyquinoline salts, 8-hydroxychinolin sulfate, salizylic acid and ist salts, 1-hydroxyethan-1,1-diphosphonic acid, tetrasodium-1-hydroxyethan-1,1-diphosphonate (CAS 3794-83-0; INCI-name TETRASODIUM ETIDRONATE), tetrasodium-imino-disuccinate (CAS 144538-83-0), ethylendiamin-tetrasodium acetate (INCI-name: EDTA) or N-(4-ethoxyphenyl)-acetamide (INCI-name: PHENACETIN)

### Hair coloring method

A hair coloring method according to the invention is a method changing the color of the hair. This comprises temporary color changes, e.g. by the uses or inorganic or organic pigments, semi-permanent color changes, e.g. by use of conventional direct dyes, permanent color changes, e.g. by use of oxidative coloring or color changes by bleaching, e.g. by use of oxidative agents. A typical hair coloring method according to the invention is the following. Freshly shampooed or wet or dry hair is parted e.g. with a comb and a hair strand held upwards. Preferred is an application on dry hair. A foam body of suitable size, length and volume is generated either directly on or near the hair line, or on or near the scalp or is generated seperately and then placed on or near the hair line, or on or near the scalp. The hair strand is positioned on top of the foam body and can optionally be modelled into the desired shape with the hand or a mechanical modelling aid such as a comb. The hair and the foam body are dried, preferably with a drying aid. The drying aid can be an electrical or electromagnetical device such as an hair drier or an infrared device. The foam body is converted to a hardened, solid structure on drying. The raised hair on top of the dry foam body stands apart from the other hair. This raised hair strand can be selectively colored or it can be left uncolored and the rest of the hair can be selectively colored or different colors or shades can be applied to one or more different raised hair strands and/or to the rest of the hair. One or more coloring compositions are applied to the raised strand, to different raised strands or to non-raised hair. The coloring compositions can be e.g. in the form of foam shades containing direct dyes or in the form of oxidative hair coloring compositions (comprising a color precursor composition containing developers and couplers and an oxidative composition containing an oxidant) or in the form of bleaching compositions such as for example bleaching gels containing a bleaching agent such as hydrogenperoxide. The coloring compositions are rinsed from the hair after an approriate treatment time. Ususally, the dry foam body is removed concomitantly during the rinsing step. If not, it can be removed by hand in one or more pieces or it is combed out by a suitable combing device such as a comb or a brush. The colored or bleached hair can optionally be treated with a conventional color saving or hair conditioning composition.

The coloring composition contain at least one hair coloring or dyeing substance. This can be for example a soluble organic dyestuff, especially a so-called direct dye compound or an inorganic or organic pigment, or a dye precursor which form a dye on oxidation. The total amount of dyestuffs or dye precursor compounds in the composition of the invention is preferably about 0.01 to 7 percent by weight, especially preferably from about 0.2 to 4 percent by weight. Suitable direct-dyeing dye compounds include e.g. triphenylmethane dye compounds, aromatic nitro dye compounds, azo dye compounds, quinone dye compounds, cationic or anionic dye compounds. Suitable hair dying pigments are coloring agents, which are practically insoluble in the application medium, and can be inorganic or organic. Also inorganic-organic mixed pigments are possible. The pigments however are preferably not nanopigments. The preferred particle size amounts to 1 to 200 µm, especially 3 to 150 µm, and even more preferably from 10 to 100 µm. Inorganic pigments are preferred. The inorganic pigments can be of a natural origin, for example chalk, ocher, umber, green earth, burnt Terra di Siena or graphite. The pigments can be white pigments, such as titanium dioxide or zinc oxide, black pigments, such as iron oxide black, colored pigments, such as ultramarine or iron oxide red, lustrous pigments, metal effect pigments, pearlescent pigments and fluorescence or phosphorescence pigments. Preferably at least one pigment is a non-white pigment. Metal oxides, metal hydroxides and metal oxihydrates, mixed phase pigments, sulfur-containing silicates, metal sulfides, complex metal cyanides, metal sulfates, metal chromates and metal molybdates, as well as metals themselves (Bronze pigments) are suitable. Especially titanium dioxide (Cl 77891), black iron oxide (Cl 77499), yellow iron oxide (Cl 77492), red and brown iron oxide (Cl 77491), mangan violet (Cl 77742), Ultramarine (Sodium aluminum sulfo silicate, Cl 77007, Pigment Blue 29), Chromium oxide hydrate (Cl 77289), iron blue (Ferric ferrocyanide, Cl 77510), Carmine (Cochineal), are suitable as the pigments in embodiments of the cosmetic preparations according to the invention. Pigments based on mica, which are coated with a metal oxide or a metal oxychloride, such as titanium dioxide or bismuth oxychloride and, if 15 necessary, other coloring agents, such as iron oxides, iron blue, Ultramarine, Carmine, etc, are particularly preferred. The colors of these particular preferred pigments are determined by variation of the coating thickness. These pigments are marketed by Merck, Germany, under the trademarks RONA®, COLORONA®, DICHRONA® and TIMIRON®. Organic pigments include, e.g., the natural pigments sepia, gamboge, animal charcoal, Kasseler brown, indigo, chlorophyll and other plant pigments. Synthetic organic pigments are, e.g., azo pigments, anthraquinoid, indigoid, dioxazine, quinacridone, phthalocyanine, isoindolinone, perylene and perinone, metal complex, alkali blue and diketopyrrolopyrrole pigments.

The coloring composition can be an oxidation dye composition. The color is generated by reaction of developers with couplers in the presence of an appropriate oxidant. The developers used for this purpose are, in particular, 2,5-diaminotoluene, 2,5-diaminophenylethyl alcohol, p-aminophenol, 1,4-diaminobenzene and 4,5-diamino-1-(2-hydroxyethyl)pyrazole, whereas suitable couplers are, for example, resorcinol, 2-methylresorcinol, 1-naphthol, 3-aminophenol, m-phenylenediamine, 2-amino-4-(2'-hydroxyethyl)aminoanisole, 1,3-diamino-4-(2'-hydroxyethoxy)benzene and 2,4-diamino-5-fluorotoluene. The couplers and developers can be contained in the coloring composition either individually or in admixture with each other, the total amount of couplers and developers in the coloring composition (based on the total amount of ready-to-use colorant) being from about 0.005 to 20 weight percent, preferably from about 0.01 to 5 weight percent and particularly from 0.1 to 2.5 weight percent, each. The total amount of the developer-coupler combination contained in the coloring composition is preferably from about 0.01 to 20 weight percent (based on the ready-to-use colorant), an amount from about 0.02 to 10 weight percent and particularly from 0.2 to 6 weight percent being especially preferred. In general, the developers and couplers are used in approximately equimolar amounts. However, it is not disadvantageous if the developers are present in a certain excess or deficiency with respect to such an amount. For use in oxidative dyeing of hair, the afore-described colorant is mixed with an oxidant just before use and the resulting mixture is applied to the hair in an amount sufficient for the hair treatment, in general in an amount from about 60 to 200 grams, depending on the fullness of the hair. Suitable oxidants for developing the hair coloration which can also be applied in bleaching compositions are mainly hydrogen peroxide or the compounds of addition thereof to urea, melamine, sodium borate or sodium carbonate in the form of a preferably 3 to 12%, more preferably 6% aqueous solution. Atmospheric oxygen can also be used. If a 3 to 6% hydrogen peroxide solution is used as oxidant, the weight ratio of hair colorant to oxidant is preferably from 5:1 to 1:5, more preferably 2:1 to 1:3 and particularly 1:1. Larger amounts of oxidant are used primarily at higher dye concentrations in the hair colorant or when more pronounced hair bleaching is wanted at the same time. In principle, adding other oxidants, for example persulfate salts is just as possible as the use of atmospheric oxygen. The mixture is allowed to act on the hair at preferably 15 to 50° C. for about preferably 10 to 45 minutes, more preferably for 10 to 20 minutes, after which the hair is rinsed with water and dried. Finally, the hair may be additionally washed with a shampoo and optionally post-rinsed with a weak organic acid, for example citric acid or tartaric acid. The hair is then dried.

### Hair care method

A hair care method according to the invention is a method comprising a step of applying at least one beneficial hair care composition to the hair. Hair care compositions containing at least one hair care agent such as hair conditioning agents which make the hair softer, improve the feel, the shine, the wet combability or the dry combability of hair or reduce the fly-away effect. A typical hair care method according to the invention is the following. Freshly shampooed or wet or dry hair is parted e.g. with a comb and a hair strand held upwards. A foam body of suitable size, length and volume is generated either directly on or near the hair line, or on or near the scalp or is generated seperately and then placed on or near the hair line, or on or near the scalp. The hair strand is positioned on top of the foam body and can optionally be modelled into the desired shape with the hand or a mechanical modelling aid such as a comb. The hair and the foam body are dried, preferably with a drying aid. The drying aid can be an electrical or electromagnetical device such as an hair drier or an infrared device. The foam body is converted to a hardened, solid structure on drying. The raised hair on the dry foam body stands apart from the other hair. Either the raised hair strand can be selectively treated with a hair care composition or it can be left untreated and the rest of the hair can be selectively treated or different hair care compositions can be applied to one or more different raised hair strands and/or to the rest of the hair. One or more hair care compositions are applied to the raised strand, to different strands or to non-raised hair. The hair care compositions can be rinsed from the hair after an approriate treatment time or they can be left on the hair. The hardened foam body is removed, either by rinsing or by hand in one or more pieces or it is combed out by a suitable combing device such as a comb or a brush.

### EXAMPLES

The compositions illustrated in the following examples illustrate specific embodiments of the cosmetic compositions of the present invention. The compositions illustrated in the following examples are prepared by conventional formulation and mixing methods. All exemplified amounts are listed as weight percents and exclude minor materials such as diluents, preservatives, color solutions, imagery ingredients, botanicals, and so forth, unless otherwise specified. If a trade name is mentioned as ingredient and the respective product is itself a mixture (e.g. a solution, emulsion, dispersion etc.), then the exemplified amount relates to this mixture, unless otherwise specified.

### Example 1

| | |
|---|---|
| 40,0 | Luviset Clear®¹ |
| 8,00 | Rewoteric® AM CAS² |
| Ad 100 | Water |

| | |
|---|---|
| ¹ INCI-name VP/Methacrylamide/Vinyl Imidazole Copolymer, available from BASF; 20% in water ² INCI-name: Cocamidopropyl Hydroxysultaine, 50% in water | |

70% by weight of the above composition is filled together with 30% by weight of a propane/butane propellant mixture (2.7 bar) into a pressurized aerosol container with foam cap.

### Example 2 A-B

| | 2A | 2B |
|---|---|---|
| Luviskol® VA 37 E³ | 20 | 20 |
| Rewoteric® AM CAS² | 5 | - |
| Genamin® CTAC 50⁴ | - | 5 |
| Water | Ad 100 g | Ad 100 g |

| | | |
|---|---|---|
| ³ INCI-name: VP/VA Copolymer, 50% in ethanol ⁴ INCI-name: Cetrimoniumchloride, 50% solution in isopropanol/water | | |

60% by weight of the above composition is filled together with 40% by weight of a propane/butane propellant mixture (1.5 bar) into a pressurized aerosol container with foam cap.

The foam compositions 1, 2A and 2B are used for increasing the volume of the hairstyle. First, hair is wetted. Optionally, the hair can additionally be treated with a conventional styling foam prior to applying the composition according to the invention. Foam is generated from examples 1, 2A and 2B directly under a strand of hair, between scalp and hair. The hair and the foam are dried with a drying device. The dried foam is removed by brushing.

### Results:

The foam structure of comparative example 2B completely disappears on drying whereas a fine, grated structure of dried foam remains after drying in example 1 and 2A. The resulting hair style of hair treated with non-dissipating foam of example 1 or 2A is of higher volume than the hair style of hair treated with dissipating foam of comparative example 2A. Hair treated with example 2A is easier brushable than hair treated with example 1. No residues remain after brushing hair treated with example 2A while some residues remain on hair treated with example 1.

The exemplified embodiments of the present invention provide treatment benefits of a more rapid, an easier and more selective over-all treatment as well as a treatment that is more comfortable and pleasant to the consumer than conventional treatments with the use of rollers, clips or pins instead of the hardened foam according to the invention when used in a hair styling method, a method for permanently shaping the hair, a method of coloring hair, or a hair care method.

## Claims

1. Cosmetic treatment method comprising
- providing a foamable composition comprising
(a) at least 1% by weight based on the composition without propellants of a zwitterionic surfactant or a surfactant mixture comprising at least one zwitterionic surfactant,
(b) at least one hair fixing polymer,
(c) water, and
(d) at least 20% by weight, based on the total composition of at least one propellant;
- forming a foam from said foamable composition on release from a pressurized container,
- drying said foam wherein the foam does not disappear upon drying,
wherein said foam is applied or generated between the scalp and scalp hair and thereby raising the hair.

2. Method according to the preceding claim wherein performing an additional hair treating step, wherein
- the method is a non-permanent hair styling method comprising the additional step of bringing the hair in a desired shape; or
- the method is a method for permanently shaping the hair, comprising the additional step of contacting the hair with a permanent wave composition comprising at least one keratin-reducing agent and/or a fixation composition containing at least one oxidising agent; or
- the method is a method of coloring hair comprising the additional step of contacting the hair with a composition containing a hair coloring agent and/or a hair bleaching agent; or
- the method is a hair care method comprising the additional step of applying a hair care active to the hair.

3. Method according to any of the preceding claims wherein the dried foam is finally removed by rinsing or by hand or by combing or brushing.

4. Use of dried foam made from a foamable composition comprising
(a) at least 1% by weight based on the composition without propellants of a zwitterionic surfactant or a surfactant mixture comprising at least one zwitterionic surfactant,
(b) at least one hair fixing polymer,
(c) water, and
(d) at least 20% by weight, based on the total composition of at least one propellant in a cosmetic treatment method of treating scalp hair or the scalp.

5. Use according to the preceding claim, wherein the dried foam is used as mechanical support for raising scalp hair.

6. Use according to the preceding use claims, wherein the dried foam is used as a substitute for hair curlers, rollers, pins, hair clips or hairgrips.

7. Device comprising
- a composition capable of forming a foam that does not disappear on drying; and
- an aerosol container equipped with a foaming device;
wherein said composition comprises
(a) at least 1% by weight based on the composition without propellants of a zwitterionic surfactant or a surfactant mixture comprising at least one zwitterionic surfactant,
(b) at least one hair fixing polymer,
(c) water, and
(d) at least 20% by weight, based on the total composition of at least one propellant.

8. Device according to the preceding claim, wherein the zwitterionic surfactant is selected from surfactants containing a betaine or a sultaine structure.

9. Device according to any of the preceding device claims, wherein the zwitterionic surfactant is selected from alkylsultaines and fatty acid amidoalkyl hydroxysultaines.

10. Device according to any of the preceding device claims, wherein the amount of zwitterionic surfactant is from 3 to 15% by weight based on the composition without propellants.

11. Device according to any of the preceding device claims, wherein the amount of hair fixing polymer is from 1 to 20% by weight based on the composition without propellants.

12. Device according to any of the preceding device claims, wherein the hair fixing polymer is selected from polyvinylpyrrolidone; copolymers of vinylpyrrolidone and vinylacetate; copolymers of vinylpyrrolidone, methacrylamide and vinylimidazole; copolymers of vinylacetate and crotonic acid; copolymers of octylacrylamide, acrylic acid, butylamino methacrylate, methyl methacrylate and hydroxypropylmethacrylate; copolymers of alkylacrylate, acrylic acid and alkylacrylamide; chitosane; methylvinyl imidazolium chloride/vinyl pyrrolidone copolymer; quaternised vinyl pyrrolidone/dimethylaminoethyl methacrylate copolymer; quaternised cellulose.

13. Device according to any of the preceding device claims, wherein the weight ratio of hair fixing polymer to surfactant is above 2:1.

14. Device according to any of the preceding device claims, wherein the amount of water is from 50 to 95% by weight based on the composition without propellants.

15. Device according to any of the preceding device claims, wherein the propellant is selected from propane, butane, isobutane, dimethylether and compressed gases or mixtures of said propellants.

16. Device according to any of the preceding device claims, wherein the composition additionally contains at least 0,01% by weight of at least one hair care active selected from silicones, cationic surfactants, light protection agents, oils, waxes, pigments, betaine, panthenol, panthenylethylether, protein hydrolysates and plant extracts.

17. Device according to any of the preceding device claims, wherein the aerosol container is equipped with an elongated nozzle.

18. Use of the device according to any of the device claims in a method according to any of the method claims.

## Patentansprüche

1. Kosmetisches Behandlungsverfahren, umfassend
- Bereitstellung einer aufschäumbaren Zusammensetzung, umfassend
(a) zu mindestens 1 Gew.-% auf Basis der Zusammensetzung ohne Treibstoffe ein zwitterionisches Tensid oder eine Tensidmischung, die mindestens ein zwitterionisches Tensid umfasst,
(b) mindestens ein Haarfixierpolymer,
(c) Wasser und
(d) zu mindestens 20 Gew.-% auf Basis der Gesamtzusammensetzung mindestens einen Treibstoff;
- Bildung eines Schaums von der aufschäumbaren Zusammensetzung bei Abgabe von einem unter Druck gesetzten Behälter,
- Trocknen des Schaums, wobei der Schaum beim Trocknen nicht verschwindet,
wobei der Schaum zwischen der Kopfhaut und dem Kopfhauthaar aufgetragen oder erzeugt wird und **dadurch** das Haar anhebt.

2. Verfahren nach dem vorstehenden Anspruch, wobei ein zusätzlicher Haarbehandlungsschritt durchgeführt wird, wobei
- das Verfahren ein nicht-permanentes Frisierverfahren ist, das den zusätzlichen Schritt des In-eine-gewünschte-Form-Bringens des Haars umfasst; oder
- das Verfahren ist ein Verfahren zur dauerhaften Formung des Haars, das den zusätzlichen Schritt des In-Berührung-Bringens des Haars mit einer permanenten Wellenzusammensetzung, die mindestens ein Keratin-Reduktionsmittel umfasst, und/oder einer Fixierzusammensetzung, die mindestens ein Oxidiermittel enthält, umfasst; oder
- das Verfahren ist ein Verfahren des Haarfärbens, das den zusätzlichen Schritt des In-Berührung-Bringens des Haars mit einer Zusammensetzung umfasst, die ein Haarfärbemittel und/oder ein Haarbleichmittel umfasst; oder
- das Verfahren ist ein Haarpflegeverfahren, das den zusätzlichen Schritt des Auftragens eines Haarpflegemittels auf das Haar umfasst.

3. Verfahren nach einem der vorstehenden Ansprüche, wobei der getrocknete Schaum schließlich durch Ausspülen oder von Hand oder durch Kämmen oder Bürsten entfernt wird.

4. Verwendung von getrocknetem Schaum aus einer aufschäumbaren Zusammensetzung, umfassend
(a) zu mindestens 1 Gew.-% auf Basis der Zusammensetzung ohne Treibstoffe ein zwitterionisches Tensid oder eine Tensidmischung, die mindestens ein zwitterionisches Tensid umfasst,
(b) mindestens ein Haarfixierpolymer,
(c) Wasser und
(d) zu mindestens 20 Gew.-% auf Basis der Gesamtzusammensetzung mindestens einen Treibstoff;

5. Verwendung nach dem vorstehenden Anspruch, wobei der getrocknete Schaum als mechanische Stütze zum Anheben des Kopfhauthaars verwendet wird.

6. Verwendung nach den vorstehenden Verwendungsansprüchen, wobei der getrocknete Schaum als ein Ersatz für Lockenwickler, Papilotten, Nadeln, Haarspangen oder Haarklammern verwendet wird.

7. Vorrichtung, umfassend
- eine Zusammensetzung, die in der Lage ist, einen Schaum zu bilden, der nicht beim Trocknen verschwindet;und
- einen Aerosolbehälter, der mit einer Schaumbildungsvorrichtung ausgestattet ist;
wobei die Zusammensetzung Folgendes umfasst
(a) zu mindestens 1 Gew.-% auf Basis der Zusammensetzung ohne Treibstoffe ein zwitterionisches Tensid oder eine Tensidmischung, die mindestens ein zwitterionisches Tensid umfasst,
(b) mindestens ein Haarfixierpolymer,
(c) Wasser und
(d) mindestens 20 Gew.-% auf Basis der Gesamtzusammensetzung mindestens einen Treibstoff;

8. Vorrichtung nach dem vorstehenden Anspruch, wobei das zwitterionische Tensid aus Tensiden ausgewählt ist, die eine Betain- oder eine Sultainstruktur enthalten.

9. Vorrichtung nach einem der vorstehenden Vorrichtungsansprüche, wobei das zwitterionische Tensid aus Alkylsultainen und Fettsäure-Amidoalkylhydroxysultainen ausgewählt ist.

10. Vorrichtung nach einem der vorstehenden Vorrichtungsansprüche, wobei die Menge des zwitterionischen Tensids von 3 bis 15 Gew.-% auf Basis der Zusammensetzung ohne Treibstoffe beträgt.

11. Vorrichtung nach einem der vorstehenden Vorrichtungsansprüche, wobei die Menge des Haarfixierpolymers von 1 bis 20 Gew.-% auf Basis der Zusammensetzung ohne Treibstoffe beträgt.

12. Vorrichtung nach einem der vorstehenden Vorrichtungsansprüche, wobei das Haarfixierpolymer von Polyvinylpyrrolidon; Copolymeren von Vinylpyrrolidon und Vinylacetat; Copolymeren von Vinylpyrrolidon, Methacrylamid und Vinylimidazol; Copolymeren von Vinylacetat und Krotonsäure; Copolymeren von Octylacrylamid, Acrylsäure, Butylaminomethacrylat, Methylmethacrylat und Hydroxypropylmethacrylat; Copolymeren von Alkylacrylat, Acrylsäure und Alkylacrylamid; Chitosan; Methylvinylimidazoliumchlorid/Vinylpyrrolidoncopolymer; quaternisiertem Vinylpyrrolidon/Dimethylaminoethylmethacrylatcopolymer; quaternisierter Cellulose ausgewählt ist.

13. Vorrichtung nach einem der vorstehenden Vorrichtungsansprüche, wobei das Gewichtsverhältnis von Haarfixierpolymer zu Tensid über 2:1 liegt.

14. Vorrichtung nach einem der vorstehenden Vorrichtungsansprüche, wobei die Menge des Wassers von 50 bis 95 Gew.-% auf Basis der Zusammensetzung ohne Treibstoffe beträgt.

15. Vorrichtung nach einem der vorstehenden Vorrichtungsansprüche, wobei der Treibstoff von Propan, Butan, Isobutan, Dimethylether und komprimierten Gasen oder Mischungen der Treibstoffe ausgewählt ist.

16. Vorrichtung nach einem der vorstehenden Vorrichtungsansprüche, wobei die Zusammensetzung zusätzlich zu mindestens 0,01 Gew.-% mindestens einen Haarpflegewirkstoff enthält, der von Siliconen, kationischen Tensiden, Lichtschutzmitteln, Ölen, Wachsen, Pigmenten, Betain, Panthenol, Panthenylethylether, Proteinhydrolysaten und Pflanzenextrakten ausgewählt ist.

17. Vorrichtung nach einem der vorstehenden Vorrichtungsansprüche, wobei der Aerosolbehälter mit einer länglichen Düse ausgestattet ist.

18. Verwendung der Vorrichtung nach einem der Vorrichtungsansprüche in einem Verfahren nach einem der Verfahrensansprüche.

## Revendications

1. Procédé de traitement cosmétique comprenant
- la fourniture d'une composition moussable comprenant
(a) au moins 1 % en poids, basé sur la composition sans propulseurs d'un agent tensioactif zwittérionique ou d'un mélange tensioactif comprenant au moins un agent tensioactif zwittérionique,
(b) au moins un polymère de fixation des cheveux,
(c) de l'eau, et
(d) au moins 20 % en poids, basé sur la composition totale, d'au moins un propulseur ;
- la formation d'une mousse à partir de ladite composition expansible à la libération depuis un récipient pressurisé,
- le séchage de ladite mousse où la mousse ne disparaît pas lors du séchage,
dans lequel ladite mousse est appliquée ou générée entre le cuir chevelu et les cheveux du cuir chevelu levant de ce fait les cheveux.

2. Procédé selon la revendication précédente, dans lequel on exécute une étape supplémentaire de traitement de cheveux, où
- le procédé est un procédé de coiffage des cheveux non permanent comprenant l'étape supplémentaire consistant à amener les cheveux dans une forme souhaitée ; ou
- le procédé est un procédé pour mettre en forme de manière permanente les cheveux, comprenant l'étape supplémentaire consistant à mettre en contact les cheveux avec une composition de permanente comprenant au moins un agent réducteur de kératine et/ou une composition de fixation contenant au moins un agent oxydant ; ou
- le procédé est un procédé de coloration des cheveux comprenant l'étape supplémentaire consistant à mettre en contact les cheveux avec une composition contenant un agent de coloration capillaire et/ou un agent d'éclaircissement des cheveux ; ou
- le procédé est un procédé de soin capillaire comprenant l'étape supplémentaire consistant à appliquer un agent actif de soin capillaire sur les cheveux.

3. Procédé selon l'une quelconque des revendications précédentes, dans lequel la mousse séchée est enfin éliminée par rinçage ou à la main ou par peignage ou brossage.

4. Utilisation d'une mousse séchée fabriquée à partir d'une composition moussable comprenant
(a) au moins 1 % en poids, basé sur la composition sans propulseurs d'un agent tensioactif zwittérionique ou d'un mélange tensioactif comprenant au moins un agent tensioactif zwittérionique,
(b) au moins un polymère de fixation des cheveux,
(c) de l'eau, et
(d) au moins 20 % en poids, basé sur la composition totale, d'au moins un propulseur dans un procédé de traitement cosmétique consistant à traiter les cheveux du cuir chevelu ou le cuir chevelu.

5. Utilisation selon la revendication précédente, dans laquelle la mousse séchée est utilisée en tant que support mécanique pour lever les cheveux du cuir chevelu.

6. Utilisation selon les revendications d'utilisation précédentes, dans laquelle la mousse séchée est utilisée en tant que substitut pour des bigoudis, rouleaux, épingles à cheveux, clips à cheveux ou pinces à cheveux.

7. Dispositif comprenant
- une composition capable de former une mousse qui ne disparaît pas au séchage ; et
- un récipient pour aérosol équipé d'un dispositif de moussage ;
dans lequel ladite composition comprend
(a) au moins 1 % en poids basé sur la composition sans propulseurs d'un agent tensioactif zwittérionique ou d'un mélange tensioactif comprenant au moins un agent tensioactif zwittérionique,
(b) au moins un polymère de fixation des cheveux,
(c) de l'eau, et
(d) au moins 20 % en poids, basé sur la composition totale, d'au moins un propulseur.

8. Dispositif selon la revendication précédente, dans lequel l'agent tensioactif zwittérionique est choisi parmi les agents tensioactifs contenant une structure bétaïne ou sultaïne.

9. Dispositif selon l'une quelconque des revendications de dispositif précédentes, dans lequel l'agent tensioactif zwittérionique est choisi parmi les alkylsultaïnes et les amidoalkyl hydroxysultaïnes d'acide gras.

10. Dispositif selon l'une quelconque des revendications de dispositif précédentes, dans lequel la quantité d'agent tensioactif zwittérionique va de 3 à 15 % en poids, basé sur la composition sans propulseurs.

11. Dispositif selon l'une quelconque des revendications de dispositif précédentes, dans lequel la quantité de polymère de fixation des cheveux va de 1 à 20 % en poids, basé sur la composition sans propulseurs.

12. Dispositif selon l'une quelconque des revendications de dispositif précédentes, dans lequel le polymère de fixation des cheveux est choisi parmi le polyvinylpyrrolidone ; des copolymères de vinylpyrrolidone et de vinylacétate ; des copolymères de vinylpyrrolidone, de méthacrylamide et de vinylimidazole ; des copolymères de vinylacétate et d'acide crotonique ; des copolymères d'octylacrylamide, d'acide acrylique, de butylamino méthacrylate, de méthacrylate de méthyle et d'hydroxypropylméthacrylate ; des copolymères d'alkylacrylate, d'acide acrylique et d'alkylacrylamide ; la chitosane ; un copolymère chlorure de méthylvinyl imidazolium/vinyl-pyrrolidone ; un copolymère de vinyl-pyrrolidone quaternaire/ méthacrylate de diméthylaminoéthyle ; une cellulose quaternaire.

13. Dispositif selon l'une quelconque des revendications de dispositif précédentes, dans lequel le rapport pondéral de polymère de fixation des cheveux sur agent tensioactif est supérieur à 2:1.

14. Dispositif selon l'une quelconque des revendications de dispositif précédentes, dans lequel la quantité d'eau va de 50 à 95 % en poids, basé sur la composition sans propulseurs.

15. Dispositif selon l'une quelconque des revendications de dispositif précédentes, dans lequel le propulseur est choisi parmi le propane, le butane, l'isobutane, le diméthyl éther et des gaz comprimés ou des mélanges desdits propulseurs.

16. Dispositif selon l'une quelconque des revendications de dispositif précédentes, dans lequel la composition contient, en outre, au moins 0,01 % en poids, d'au moins un agent actif de soin capillaire choisi parmi les silicones, agents tensioactifs cationiques, agents de protection contre la lumière, huiles, cires, pigments, bétaïne, panthénol, panthényléthyléther, hydrolysats de protéines et extraits de plantes.

17. Dispositif selon l'une quelconque des revendications de dispositif précédentes, dans lequel le récipient pour aérosol est équipé d'une buse allongée.

18. Utilisation du dispositif selon l'une quelconque des revendications de dispositif précédentes, dans un procédé selon l'une quelconque des revendications de procédé.
